# EUROPÄISCHE PATENTSCHRIFT

(11) **EP 3 641 950 B1**
(45) Veröffentlichungstag und Bekanntmachung des Hinweises auf die Patenterteilung: **07.06.2023**
(21) Anmeldenummer: 18735494.9
(22) Anmeldetag: 19.06.2018
(51) Int. Cl.: B05D 5/08, A61L 27/34, C08G 79/025, A61L 27/04, A61L 27/56, A61L 33/06, C09J 185/02, B05D 1/18, B82Y 30/00

(54) **BESCHICHTUNG ODER MIT EINER BESCHICHTUNG VERSEHENER KÖRPER SOWIE VERFAHREN ZU DEREN HERSTELLUNG**
COATING OR BODY PROVIDED WITH A COATING, AND METHOD FOR PRODUCING SAME
REVÊTEMENT OU CORPS REVÊTU ET PROCÉDÉ POUR SA FABRICATION

(30) Priorität: 21.06.2017 DE 102017113758
(43) Veröffentlichungstag der Anmeldung: 29.04.2020
(73) Patentinhaber: Universität des Saarlandes, 66123 Saarbrücken (DE)
(72) Erfinder: HAIDAR, Ayman, 66125 Saarbrücken (DE); VEITH, Michael, 66386 St. Ingbert (DE); AKTAS, Cenk, 24143 Kiel (DE); ABDUL-KHALIQ, Hashim, 66424 Homburg (DE); AWADELKAREEM, Ali A., 66123 Saarbrücken (DE)
(74) Vertreter: Wolff, Matthias
(86) Internationale Anmeldenummer: PCT/DE2018/100571
(87) Internationale Veröffentlichungsnummer: WO 2018/233767

(56) Entgegenhaltungen:
- CN-B- 104 549 159
- DE-A1-102010 027 063
- Felix Zeifang ET AL: "Improved osseointegration of PTFEP-coated titanium implants", Medical science monitor : international medical journal of experimental and clinical research, 1. Februar 2008 (2008-02-01), Seiten BR35-BR40, XP055505737, United States Gefunden im Internet: URL:https://www.medscimonit.com/download/i ndex/idArt/734747 [gefunden am 2018-09-10]
- Mario Gleria ET AL: "Phosphazenes and Surfaces" In: "Polyphosphazenes for Biomedical Applications", 10. November 2008 (2008-11-10), John Wiley & Sons, Inc., XP055505716, ISBN: 978-0-470-47888-2 Seiten 185-224, DOI: 10.1002/9780470478882.ch12, Seite 204, Absatz 2 - Seite 207, Absatz 1
- JIANWEI FU ET AL: "Preparation of Silver Nanocables Wrapped with Highly Cross-Linked Organic-Inorganic Hybrid Polyphosphazenes via a Hard-Template Approach", JOURNAL OF PHYSICAL CHEMISTRY C, Bd. 112, Nr. 43, 8. Oktober 2008 (2008-10-08), Seiten 16840-16844, XP055505705, ISSN: 1932-7447, DOI: 10.1021/jp8063855

## Beschreibung

Die Erfindung betrifft eine Beschichtung oder mit einer Beschichtung versehene. Körper, wobei die Beschichtung zumindest teilweise aus einem Verbundwerkstoff gebildet ist, welcher einen porösen Träger umfasst, der einen metallischen Kern, welcher mit einem Metalloxid ummantelt ist und an dem ein Polyphosphazen gebunden ist, aufweist.

Die Erfindung betrifft ferner ein Verfahren zur Herstellung der Beschichtung oder des Körpers.

Beschichtungen oder Körper der eingangs genannten Art sind bekannt aus der Veröffentlichung Felix Zeifang et al., "Improved osseointegration of PTFEP-coated titanium implants", MEDICAL SCIENCE MONITOR, 20080201 MEDISCIENCE PUBLIKACJE NAUKOWE, WARZAW, PL; Vol:14,Nr:2,Page(s):BR35 - BR40, https://www.medscimonit.com/download/index/idArt/734747.

Weitere Beschichtungen oder Körper sind beispielsweise aus der EP 1 996 753 A1 bekannt, in der eine Kompositstruktur beschrieben ist. Die Kompositstruktur weist eine verzweigte Struktur Nanodrähten auf, welche einen metallischen Kern, der mit einem Metalloxid ummantelt ist, umfasst.

Weitere Veröffentlichungen sind die DE102010027063, die CN104549159 sowie "Phosphazenes and Surfaces" von Mario Gleria et al., Polyphosphazenes for Biomedical Applications, 20090715 John Wiley & Sons, Inc., p. 185 - 224.

Der Erfindung liegt die Aufgabe zugrunde, weitere Verwendungsmöglichkeiten für die eingangs genannte Beschichtung bzw. den Körper zu erschließen. Erfindungsgemäß wird diese Aufgabe dadurch gelöst, dass der Träger eine Vielzahl von Nanodrähten aufweist.

Überraschend hat sich gezeigt, dass Polyphosphazene sich unter Ausbildung einer chemischen, insbesondere kovalenten, Bindung mit dem Metalloxid fest an der Träger binden lassen. Die Bindung bildet sich dabei als Me-O-P-Bindung aus, wobei Me das Metall des Metalloxids, O der Sauerstoff des Metalloxids und P ein Phosphor des Polyphosphazens ist. Die Eigenschaften der Oberfläche des Trägers, insbesondere die Hydrophilie oder Hydrophobie aber auch die ölabweisenden Eigenschaften, lassen sich dadurch erheblich beeinflussen.

Als besonders vorteilhaft hat sich überraschend ferner gezeigt, dass sich durch die Beschichtung die Thrombozytenadhäsion, die Thrombozytenaktivierung, die Adhäsion von Bakterien und die Proteinadsorption im Vergleich zu den bekannten Beschichtungen erheblich verringern lässt. Aufgrund der guten Bioverträglichkeit der Polyphosphazene eignet sich der erfindungsgemäße Körper insbesondere für biomedizinische Anwendungen. Beispielsweise können die Polyphosphazene als makromolekularer Wirkstoffträger oder als biostabile oder biologisch abbaubare Gerüstmaterialien verwendet werden.

Darüber weist die Beschichtung eine außergewöhnlich große Hydrophobie auf, sodass sich die Beschichtung für diverse Anwendungen, in denen wasserabweisende Eigenschaften gefordert sind, eignet, beispielsweise für selbstreinigende Oberflächen von Fensterscheiben oder Sanitärwaren.

In einer Ausgestaltung der Erfindung umfasst oder ist das Polyphosphazen ein Fluoro-Alkoxy-Phosphazen, vorzugsweise Poly-Trifluoroethoxy-Phosphazene (PTFEP).

In einer Ausführungsform der Erfindung bilden die, vorzugsweise untereinander vernetzten, Nanodrähte gemeinsam eine poröse Struktur.

Die Nanodrähte weisen in einer Ausführungsform der Erfindung Durchmesser auf, die kleiner als 200 nm sind und vorzugsweise von 10 bis 100 nm, besonders bevorzugt
20 bis 40 nm, betragen. Die Länge der Nanodrähte beträgt zweckmäßigerweise im Mikrometer- und Submikrometerbereich. Der Querschnitt der Nanodrähte ist in der Regel annähernd kreisförmig.

In einer Ausgestaltung der Erfindung weist die Beschichtung die in der EP 1 996 753 B1 beschriebenen Nanodrähte auf. Solche Nanodrähte umfassen in einer Ausführungsform der Erfindung einen metallischen Kern, der von einem Metalloxid ummantelt ist. Der metallische Kern ist zweckmäßigerweise aus einem Metall, insbesondere aus einem Metall der III. Hauptgruppe, gebildet. Besonders gut eignen sich beispielsweise Aluminium, Gallium, Indium oder/und Thallium, wobei Aluminium oder Gallium besonders bevorzugt sind.

Eine Ummantelung des Kerns ist zweckmäßigerweise aus einem Metalloxid und insbesondere einem Metalloxid eines Metalls der III. Hauptgruppe, wie Aluminium, Gallium, Indium oder/und Thallium gebildet, wobei Aluminiumoxid oder/und Galliumoxid besonders bevorzugt werden. In einer bevorzugten Ausführungsform sind sowohl das Metall des Kerns als auch das Metall des Metalloxids ein Metall der III. Hauptgruppe, wobei das Metall des Kerns und das Metall des Metalloxids vorzugsweise dasselbe Metall der III. Hauptgruppe sind.

Das Metalloxid kann jedes übliche Oxid sein. Es umfasst Oxide in allen üblichen Oxidationsstufen der Metalle und auch Mischoxide, in denen das Metall in unterschiedlichen Oxidationsstufen enthalten ist. Die Metalloxide können amorph oder/und kristallin vorliegen. Die kristallinen Metalloxide können alle üblichen Kristallformen, beispielsweise in alpha-Al₂O₃, gamma-Al₂O₃, alpha-Ga₂O₃, beta-Ga₂O₃ und In₂O₃ aufweisen.

Gegebenenfalls können der Kern und/oder die Ummantelung geringe Mengen an Verunreinigungen enthalten, z.B. Zwischenprodukte oder Nebenprodukte aus den Ausgangsverbindungen. Die Kompositstrukturen sind aber bevorzugt im wesentlichen frei von Verunreinigungen. Insbesondere enthalten sie keine Rückstände, die von Katalysatoren oder Templatmaterialien stammen.

Während die Nanodrähte eine einfache, lineare, kabelartige und/oder eindimensionale Strukturen aufweisen kann, umfassen sie in einer besonders bevorzugten Ausgestaltung der Erfindung alternativ oder zusätzlich eine oder mehrere, ggf. untereinander, verzweigte Strukturen, die aus mehreren, ggf. astartig, aufeinander aufgewachsenen Nanodrähten der linearen Form gebildet sind. Bei der verzweigten Form können sich die metallischen Kerne der Drähte an den Verzweigungen berühren oder die Metallkerne können an den Verzweigungen durch die Metalloxidhülle voneinander getrennt sein. Während es vorstellbar wäre, dass die Nanodrähte frei vorliegen, sind sie in der bevorzugten Ausgestaltung der Erfindung direkt auf dem Körper oder auf einem geeigneten Substrat angeordnet, welches mit dem Körper verbunden ist.

In einer Ausführungsform der Erfindung werden die Nanordrähte mittels eines CVD-Verfahrens, vorzugsweise mittels eines Metal Organic Chemical Vapor Deposition-Verfahrens (MO-CVD-Verfahren) oder eines Initiated Chemical Vapor Deposition - Verfahrens (iCVD-Verfahren), hergestellt werden. Verfahren zur Herstellung der Nanodrähte mittels MO-CVD sind beispielsweise in der EP 1 996 753 A1 beschrieben.

Mittels des MO-CVD-Verfahrens können metallorganische Verbindungen, die als Vorläufer (Precursoren) dienen, zu dünnen Schichten keramischer Materialien mit sehr hoher Qualität umgesetzt werden. Beim MO-CVD-Verfahren werden die metallorganischen Verbindungen chemischen aufgedampft. Dazu ist eine hinreichende Flüchtigkeit des Precursors unter Reaktionsbedingungen erforderlich, wobei aber für die Prozesssteuerung umgekehrt eine gewisse Stabilität dieser Verbindung unter Normalbedingungen notwendig ist. Entsprechende Precursoren zur Erzeugung von flächigen Mikro- und Nanokompositen sind z.B. aus DE-A-4221659 bekannt.

Bei dem erfindungsgemäßen Verfahren wird eine metallorganische Verbindung unter Bildung der Nanodrähte thermolytisch zersetzt. Es handelt sich um ein katalysatorfreies und templatfreies Verfahren. Die verwendeten metallorganischen Verbindungen besitzen folgende allgemeine Formel: El(OR)H₂, worin El die Elemente Al, Ga, In oder TI bedeutet, wobei Al und Ga bevorzugt sind, und R für einen aliphatischen oder alicyclischen Kohlenwasserstoffrest steht.

Der aliphatische oder alicyclische Kohlenwasserstoffrest ist zweckmäßigerweise ungesättigt. Er kann z.B. 1 bis 15 C-Atome aufweisen. Bevorzugt ist Alkyl oder unsubstituiertes oder Alkyl-substituiertes Cycloalkyl. Der Alkylrest besitzt vorzugsweise
2 bis 12 C-Atome, bevorzugt 3 bis 10 C-Atome. Das Alkyl kann linear oder verzweigt sein, wobei verzweigte Alkylreste bevorzugt sind. Beispiele sind Ethyl, n-Propyl, n-Butyl und die entsprechenden höheren linearen Homologe, Isopropyl, sek.-Butyl, tert.-Butyl, Neopentyl, Neohexyl und die entsprechenden höheren Isoalkyl- und Neoalkylhomologe oder 2-Ethylhexyl. Die alicyclischen Reste können einen, zwei oder mehr Ringe umfassen, die jeweils mit Alkyl substituiert sein können. Der alicyclische Rest umfasst zweckmäßigerweise 5 bis 10 und insbesondere 5 bis 8 Kohlenstoffatome. Beispiele für geeignete Cycloalkyle sind Cyclopentyl, Cyclohexyl, Methylcyclohexyl, Norbornyl und Adamantyl.

Besonders geeignet sind Aluminiumalkoxydihydride, die verzweigte C4-C8-Alkoxyreste aufweisen, insbesondere Aluminium-tert.-butoxydihydrid. Die Herstellung von Verbindungen der allgemeinen Formel El(OR)H₂ ist bekannt und wird in DE-A-19529241 beschrieben. Sie können z.B. durch Umsetzung des El-hydrids mit dem entsprechenden Alkohol im Molverhältnis 1:1 erhalten werden, wobei das Elhydrid in situ durch Reaktion eines Alkali-El-hydrids mit EI-halogenid hergestellt werden kann. Verbindungen der Formel El(OR)H₂, worin EI Al oder Ga ist, und deren Herstellung werden auch in M. Veith et al., Chem. Ber. 1996, 129, 381-384, beschrieben. In diesem Artikel wird auch gezeigt, das die Verbindungen der Formel El(OR)H2 auch dimere Strukturen umfassen können.

Die metallorganischen Verbindungen werden in die Gasphase überführt und thermolytisch zersetzt, wobei das nichtflüchtige Zersetzungsprodukt in der Regel an oder auf einem Substrat in Form der Nanodrähte gebildet wird. Für den Körper bzw. das Substrate, auf dem die Beschichtung angeordnet wird, kommen prinzipiell alle üblichen Materialien in Betracht, z.B. Glas, Keramik, Metall, Silizium oder Quarz, die bevorzugt inert gegenüber den Ausgangs- und Endprodukten sind. Die Thermolyse kann z.B. in einem Ofen, an einer induktiv beheizten Oberfläche oder an einer auf einem induktiv geheizten Probenträger befindlichen Oberfläche durchgeführt werden. Als Substratmaterialien für die direkte induktive Heizung kommen lediglich elektrisch leitfähige Materialien, wie Graphit oder Metalle, in Frage. Bei der Verwendung von Substraten mit geringerer elektrischer Leitfähigkeit wie Glas, Quarz, Silizium, Aluminiumoxid muss ein Ofen oder, im Falle der induktiven Heizung, ein elektrisch leitender Substratträger, z.B. aus Graphit oder Metall, verwendet werden. Das Substrat kann somit eine Oberfläche des Reaktionsraums oder ein darin platziertes Substrat, z.B. ein Plättchen oder eine Folie, sein.

Der eingesetzte Reaktorraum kann jede beliebige Gestalt aufweisen. Es kann sich z.B. um ein Reaktionsrohr handeln. Als Reaktormaterial kann jedes übliche inerte Material verwendet werden, z.B. Duran- oder Quarzglas. Es versteht sich, dass der zur Durchführung des Verfahrens eingesetzte Reaktor an den jeweiligen Körper oder das jeweilige Substrat bzw. die Größe des Substrates anzupassen ist. Beispielsweise kann für ein rundes Substrat mit einem Durchmesser von 2,5 cm ein Reaktordurchmesser von 5 cm und für ein Substrat mit einem Durchmesser von 50 cm ein Reaktordurchmesser von 55 cm eingesetzt werden.

Die Erzeugung der erfindungsgemäßen Nanodrähte hängt wesentlich von der verwendeten Temperatur für die thermolytische Zersetzung und damit von der Substrattemperatur ab. Die gewünschten Strukturen entstehen bei erhöhten Temperaturen größer 400 °C, bevorzugt bei Temperaturen von mindestens 450 °C. Bevorzugt sind Temperaturen von nicht mehr als 1200 °C und insbesondere nicht mehr als 600 °C, z.B. von 400 °C bis 1200 °C und vorzugsweise von 450 bis 650 °C, am meisten bevorzugt von 450 °C bis 600 °C verwendet. Der Körper bzw. das Substrat, auf bzw. an dem die Thermolyse stattfindet, wird dementsprechend auf die gewünschte Temperatur erhitzt. Die Erzeugung der erfindungsgemäßen Strukturen ist dabei unabhängig vom verwendeten Substratmaterial und dessen Beschaffenheit.

Die metallorganische Verbindung bzw. der Precursor wird zweckmäßigerweise aus einem Vorratsgefäß, das bevorzugt auf eine gewünschte Verdampfungstemperatur temperiert ist, in den Reaktor eingeleitet. Das Vorratsgefäß kann z.B. auf eine Verdampfungstemperatur im Bereich von -50 °C bis 120 °C, vorzugsweise von -10 °C bis 40 °C temperiert werden. Die Thermolyse im Reaktorraum erfolgt in der Regel bei Atmosphärendruck oder Unterdruck, bevorzugt bei einem Vakuum von in der Regel 10⁻⁶ mbar bis Atmosphärendruck und bevorzugter im Bereich von 10⁻² mbar bis 10⁻¹ mbar. Zur Erzeugung eines Vakuums kann ausgangsseitig ein Vakuumpumpensystem an den Reaktor angeschlossen werden. Es können alle üblichen Vakuumpumpen verwendet werden, bevorzugt ist eine Kombination aus Drehschieberpumpe und Turbomolekularpumpe oder eine Drehschieberpumpe. Zweckmäßigerweise ist auf einer Seite des Reaktorraums das Vorratsgefäß für den Precursor angebracht und auf der anderen Seite das Vakuumpumpensystem.

Vor der Durchführung des Verfahrens kann der Reaktorraum durch ein- oder mehrfaches Durchspülen von Inertgas, wie Stickstoff oder Argon, gegebenenfalls unter zwischenzeitlichem Anlegen eines Vakuums, inertisiert werden. Nach Einstellen der gewünschten Bedingungen wird der Precusor über einen gewissen Zeitraum in den Reaktor eingeleitet, so dass er an bzw. auf dem Substrat unter Bildung der eindimensionalen Kompositstruktur thermolytisch zersetzt wird.

Bei Erhitzung des Substrats durch Induktion können z.B. quadratzentimetergroße, elektrisch leitende Metallplättchen oder -folien als Substrat in einem Reaktionsrohr aus Duran- oder Quarzglas angeordnet werden. An dem Reaktionsrohr sind eingangsseitig das auf die gewünschte Verdampfungstemperatur temperierte Vorratsgefäß mit dem Precursor und ausgangsseitig ein Vakuumpumpensystem angeschlossen. Das Reaktionsrohr befindet sich in einem Hochfrequenzinduktionsfeld, mit dessen Hilfe die Substratplättchen oder -folien auf die gewünschte Temperatur erhitzt werden. Nach Einstellen des gewünschten Drucks und Einleiten des Precursors wird das Substrat mit den eindimensionalen Strukturen bedeckt.

Die genaue morphologische Ausprägung der erfindungsgemäßen eindimensionalen Strukturen kann durch geeignete Auswahl der Parameter des Verfahrens gesteuert werden. Durch gezielte Wahl von Substrattemperatur und Precursorfluss (Menge an eingeleitetem Precursor pro Zeiteinheit), welcher wiederum durch gezielte Einstellung der Precursorvorlagentemperatur und der Wahl des Druckes im Reaktor steuerbar ist, können Länge, Dicke und/oder Verzweigung der Strukturen beeinflusst werden.

Um eine besonders homogene Beschichtungsstruktur zu erhalten, wird, die Abscheidungsrate auf < 10 Å/sec, vorzugsweise < 5 Å/sec, besonders bevorzugt 2 - 3 Å/sec eingestellt, um zu vermeiden, dass sich im am Substrat bzw. dem Körper Temperaturgradienten ausbilden.

In einer besonders bevorzugten Ausgestaltung der Erfindung belegt das Polyphosphazen den Träger, insbesondere die Nanodrähte, mit einer derart dünnen Schicht belegt, dass zumindest an der Oberfläche der Beschichtung, insbesondere der frei bleibenden Oberfläche der Beschichtung, die durch die Nanodrähte gebildete Struktur erhalten bleibt.

Dazu hat es sich als geeignet erwiesen, dass Polyphosphazen, insbesondere das Poly-Bis-Trifluoroethoxy-Phosphazen, in einer Konzentration von 0,2 bis 3,5 mg/cm³, besonders bevorzugt in einer Konzentration von 0,5 bis 2,5 mg/cm³, aufzubringen. Das Polyphosphazen ist zweckmäßigerweise in Aceton oder Tetrahydrofuran gelöst. Es versteht sich, dass je nach Verfahren zur Ausbildung der Schicht aus dem Polyphosphazen auch andere Lösungsmittel verwendet werden können.

In einer Ausführungsform der Erfindung wird das Polyphosphazen, vorzugsweise das Fluoro-Alkoxy-Phosphazen, insbesondere das Poly-Bis-Trifluoroethoxy-Phosphazen, mittels Dip-Coating auf den Träger, insbesondere die Nanodrähte, aufgebracht. Das Polyphosphazen liegt beim Dip-Coating vorzugsweise in der obengenannten Konzentration vor. Aceton oder Tetrahydrofuran eignen sich besonders gut für ein solches Dip-Coating.

Beim Dip-Coating wird die nanoporöse Struktur, die durch die Nanodrähte gebildet wird, durch das Polyphosphazen infiltriert.

Es versteht sich, dass neben dem Dip-Coating auch andere Verfahren zur chemischen Lösungsabscheidung, z.B. Besprühen, Bedrucken, Spin-Coating, verwendet werden können, um das Polyphosphazen auf den Träger aufzubringen.

Beim Belegen des Trägers läuft folgende Reaktion ab:

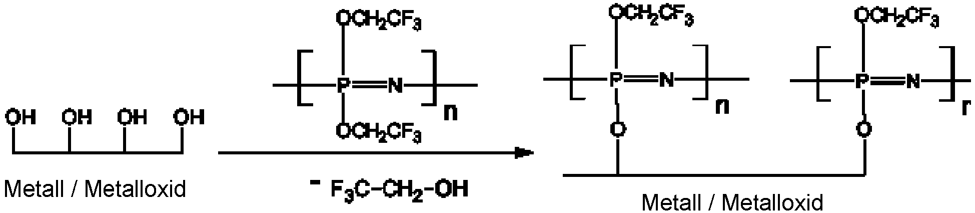

Eine mit OH-Gruppen belegte Oberfläche des Trägers reagiert mit dem Poly-Bis-Trifluoroethoxy-Phosphazen unter Abtrennung von F₃C-CH₂-OH und Ausbildung einer kovalenten Bindung zwischen der Metalloxidoberfläche.

Da für die Reaktion kein Katalysator verwendet werden muss, wird vorteilhaft eine Verunreinigug durch Katalysatorrückständen, insbesondere Katalysatorionen, vermieden, die bei der Verwendung der Beschichtung für Implantate eine toxische Wirkung entfalten können.

Da die gebildete Verbindung besonders stabil ist, wird das Polyphosphazen auch mechanisch fest an den Träger gebunden. Deshalb weist die gesamte Beschichtung eine besonders große mechanische Stabilität aus.

Nach dem Dip-Coating wird die Beschichtung bzw. der beschichtete Köper zweckmäßigerweise getrocknet, beispielsweise über einen Zeitraum von mindestens 6 Stunden, vorzugsweise mindestens 10 Stunden bei mindestens 30 °C, vorzugsweise mindestens 40 °C, getrocknet.

In einer weiteren Ausgestaltung der Erfindung ist der Körper, auf dem die Beschichtung angeordnet ist, aus einem Metall, einem Kunststoff, einer Keramik und/oder einem Glas gebildet ist. Als der Körper kommen alle Gegenstände in Betracht, für die hydrophobe, olephobe oder entzündungshemmende Eigenschaften vorteilhaft sind. In der bevorzugten Ausführungsform der Erfindung ist der Körper ein Implantat. Die Beschichtung eignet sich insbesondere zur Verwendung auf einem Stent, einer Herzklappe, einem Shunt, einem Okkluder oder einer Gefäßprothese. Es wäre aber auch vorstellbar, sie für ein Gelenkersatzimplantat, ein Zahn- und/oder Kieferimplantat, einen Herzschrittmacher oder dergleichen vorzusehen. Ferner käme infrage, die Beschichtung auf einer Glasscheibe, vorzugsweise einer Fensterscheibe, einem Sanitärobjekt, vorzugsweise einem WC-Becken, einem Urinal, einer Badewanne oder einer Duschtasse, vorzusehen, um einen schmutzabweisenden Effekt zu erzielen.

Weitere Beispiele für den Körper sind Baumaterialien, Maschinengehäuse und Fahrzeug karosserien.

Die Erfindung wird nachfolgend anhand eines Beispiels und der beigefügten Zeichnungen, Grafiken und Aufnahmen, die sich auf das Beispiel beziehen, näher erläutert Es zeigen:
- Fig. 1: REM-Aufnahmen einer erfindungsgemäßen Beschichtung,
- Fig. 2: Spektren von XPS-Analysen einer erfindungsgemäßen Beschichtung,
- Fig. 3: Spektren von NMR-Analysen einer erfindungsgemäßen Beschichtung,
- Fig. 4: Ergebnisse von Kontaktwinkelmessungen mit Wasser auf einer erfindungsgemäßen Beschichtung und verschiedenen Vergleichsoberflächen,
- Fig. 5: Ergebnisse von Messungen von Thrombozytenadhösion auf einer erfindungsgemäßen Beschichtung und verschiedenen Vergleichsoberflächen,
- Fig. 6: Ergebnisse von Messungen von der Aktivierung von Thrombozyten auf einer erfindungsgemäßen Beschichtung und verschiedenen Vergleichsoberflächen,
- Fig. 7: Ergebnisse von Messungen der Zytotoxizität auf einer erfindungsgemäßen Beschichtung und verschiedenen Vergleichsoberflächen,
- Fig. 8: Ergebnisse von Messungen der Adhäsion von Bakterien auf einer erfindungsgemäßen Beschichtung und verschiedenen Vergleichsoberflächen, und
- Fig. 9: REM-Aufnahmen von Proteinadhäsion auf einer erfindungsgemäßen Beschichtung und verschiedenen Vergleichsoberflächen

### Beispiel

Eine Glasscheibe wurde in einer Apparatur beschichtet, die aus einem Glasrohr mit zylindrischem Querschnitt (Durchmesser 4 cm, Länge ca. 50 cm) besteht. Auf einer Seite des Rohres ist ein Vakuumpumpensystem (Drehschieberpumpe und Druckmessgerät) angeschlossen. Auf der entgegengesetzten Seite des Glasrohres ist ein Vorratsgefäß angeschlossen, das mit dem Precursor Aluminium-tert.-butoxydihydrid gefüllt ist. Die Beheizung erfolgte induktiv über eine Kupferumwicklung des Reaktionsrohres. Als Unterlage für die Glasscheibe, auf der der Precursor abgeschieden werden soll, dient ein Graphitblock. Die Temperaturkontrolle erfolgte bei dieser Anordnung über ein Thermoelement. In dem Experiment wird der Graphitblock auf 600 °C erhitzt. Aluminium-tert.-butoxydihydrid wurde 30 min lang aus dem Vorratsgefäß bei 0 °C in den Reaktionsraum überführt und dort unter Bildung von mehreren 100 nm langen Aluminium-Aluminiumoxid-Nanodrähten zersetzt, die einen Durchmesser von etwa 30 nm aufweisen.

Die beschichtete Glasscheibe wurde anschließend per Dip-Coating in einer Aceton-Lösung mit 1,5 mg/cm³ Poly-Bis-Trifluoroethoxy-Phosphazen (PTFEP) beschichtet und die beschichtete Glasscheibe anschließend 12 Stunden bei 40 °C getrocknet.

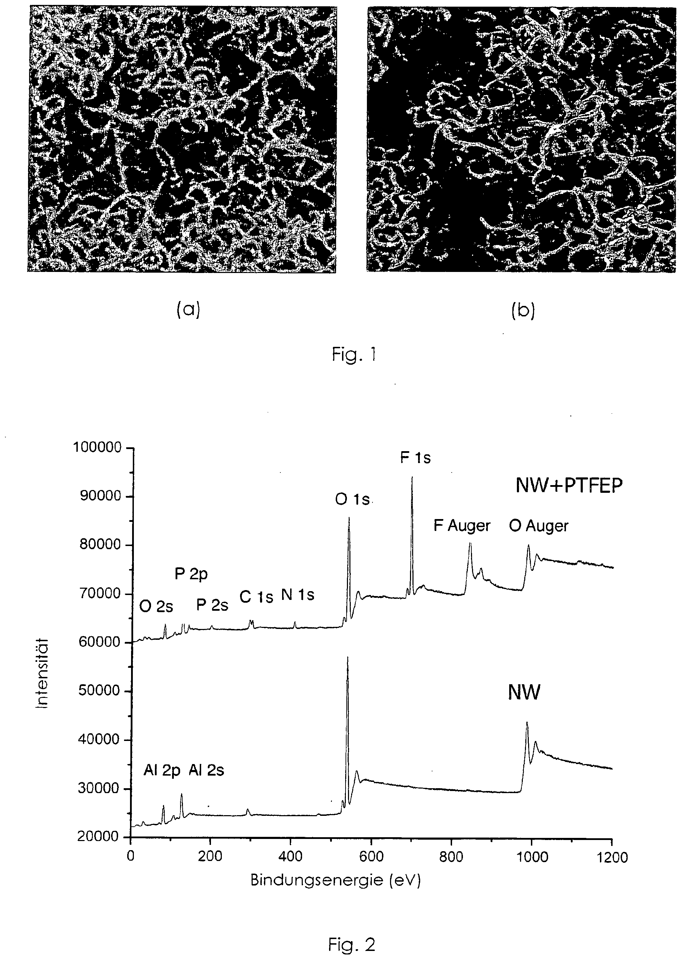

Fig. 1a zeigt eine REM-Aufnahme der gebildeten Nanodrähtebeschichtung vor dem Dipcoating und Fig. 1b eine REM-Aufnahme der gebildeten Nanodrähtebeschichtung nach dem Dipcoating und der Trocknung. Wie die Aufnahmen erkennen lassen, bleibt die Struktur der Beschichtung erhalten.

Fig. 2 zeigt Spektren von XPS-Analysen der Beschichtung vor (NW) und nach dem Dip-Coating und der Trockung (NW+PTFEP). Die Al-Peaks und die P2p und F1s-Peaks, die nach dem Dip-Coating gemessen worden sind, weisen auf eine sehr dünne Schicht aus PTFEP auf den Nanodrähten hin.

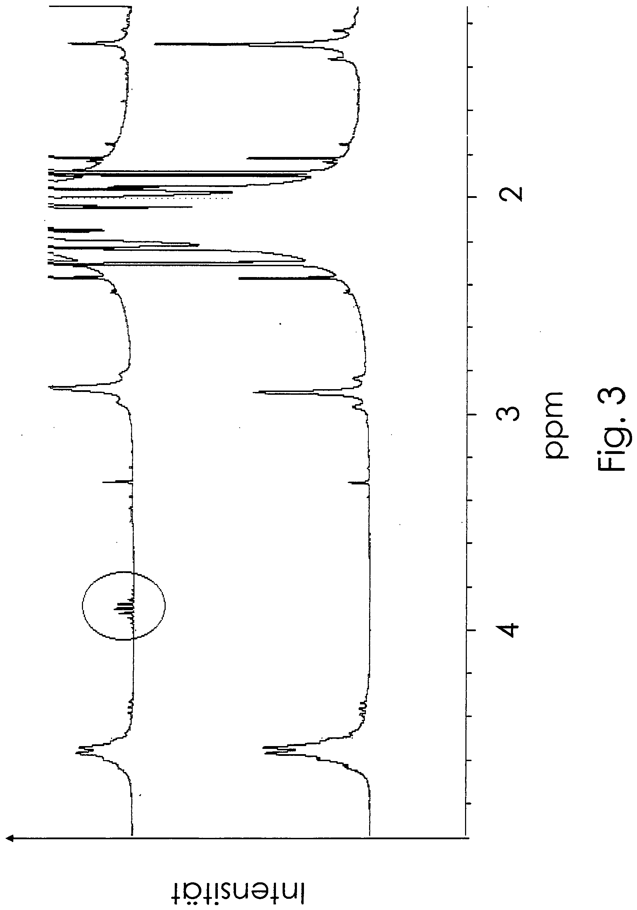

Fig. 3 zeigt NMR-Spektren der PTFEP-Lösung, die zum Dip-Coating verwendet worden ist, vor und nach dem Dip-Coating. Nach dem Dip-Coating bilden sich die im Kreis hervorgehobenen Peaks aus, die dem Trifluoroethanol (F₃C-CH₂-OH) zuzuordnen sind. Auch dies ist ein Hinweis auf die chemische Bindung des PTFEP an die Nanodrähte. Ferner zeigt der Peak bei ∂-3,9, der dem freigewordenen Trifluoroethanol (F₃C-CH₂-OH) zugeordnet werden kann, dass das PTFEP chemisch an die Nanodrähte gebunden worden ist.

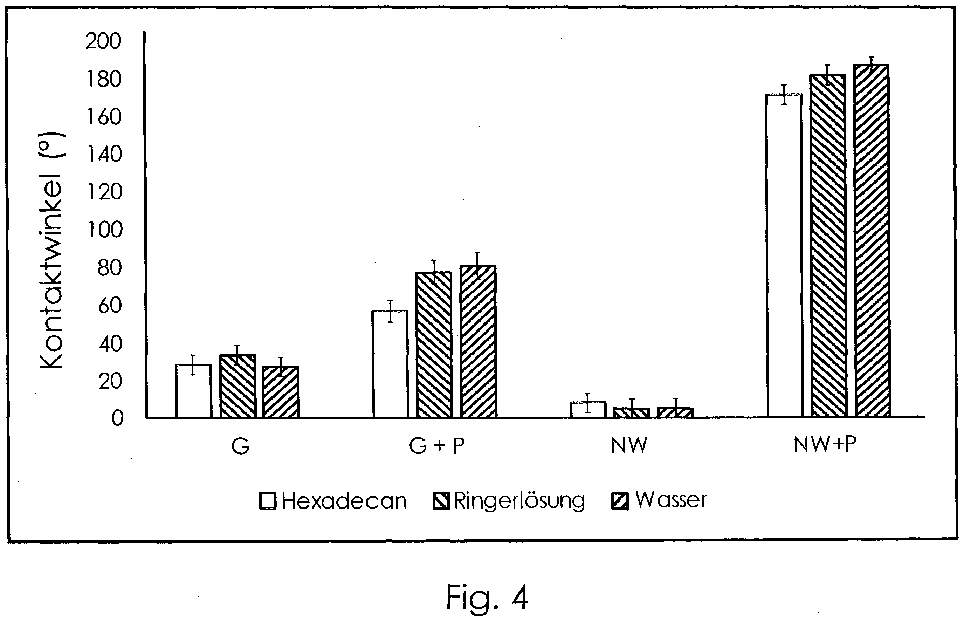

In Fig. 4 sind die Ergebnisse von Kontaktwinkelmessungen mit Wasser auf einer Oberfläche einer unbeschichteten Glasscheibe (G), einer direkt mit PTFEP mittels Dip-Coating beschichteten Glasscheibe (G + P), einer lediglich mit den Nanodrähten beschichteten Glasscheibe (G + NW) und einer erfindungsgemäßen, mit den Nanodrähten und PTFEP beschichteten Glasscheibe (G + NW + P) dargestellt. Wie der Grafik zu entnehmen ist, haben sich bei der erfindungsgemäß beschichteten Glasscheibe Kontaktwinkel nach 180 ° ausgebildet. Dieser sind wesentlich größer als bei den Vergleichsscheiben.

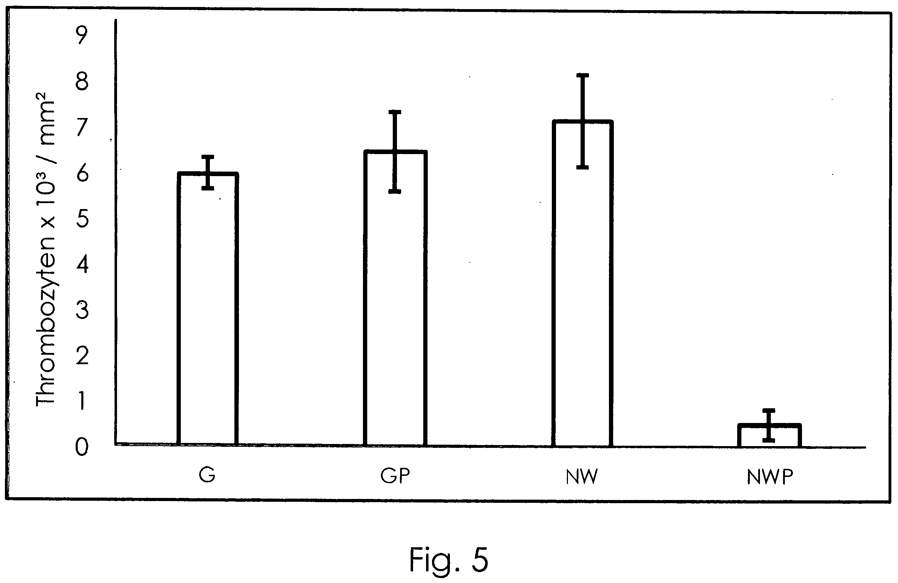

Zur Durchführung der Messungen von Thombozytenadhäsion auf der erfindungsgemäßen Beschichtung (vgl. Ergebnis in Fig. 5) wurden wie oben zu Fig. 4 erläutert, unterschiedlich beschichtete Glasscheiben in einer Vorrichtung angeordnet, mittels derer über die Oberfläche der Glasscheiben menschliches Blut geleitet werden kann. Nachdem bei 25° C während 10 Min. das Blut über die jeweilige Oberfläche geleitet worden ist, sind die Glasscheiben direkt mit DPBS (Dulbecco's-Phosphate-Buffered Saline)(GIBCO^{®}) drei mal gespült worden und danach mit frisch zubereitetem 2,5% Glutteraldehyde in 0,1 M Cacodylsäurepufferlösung jeweils drei mal für 10 Min. gewaschen und anschließend während einer Stunde bei Raumtemperatur in 1% Osmiumtetroxid (OsO₄) in 0,2 M Cacodylsäurepufferlösung zur Kontrastierung in einer Dunkelkammer inkubiert worden. Anschließend wurden die Glasscheiben zur Entfernung von Verunreinigungen mit Osmiumtextroxid mit destilliertem Wasser vier mal je 10 Min. lang gewaschen und anschließend mit einer Serie von 70%, 80%, 90%, 96% und 100% Ethanollösungen jeweils 10 Min. dehydriert und danach unter Benutzung von verschiedenen Hexamethyldisalazan (HMDS)-Lösungen getrocknet. Anschließend wurden die Proben mit reinem HMDS belegt und über Nacht unter einem Abzug angeordnet. Zur Analyse wurden die Proben mit Platin/Gold gesputtert und einem Rasterelektronenmikroskop gescannt.

Von jeder Probe wurden 10 Rasterelektronenmikroskopaufnahmen mit einer 2000-fachen Vergrößerung gemacht und die Anzahl der Thrombozyten mittels des Programm "Image J" gezählt worden sind. Die durchschnittliche Anzahl pro mm² wurde berechnet und ist in Fig. 5 dargestellt.

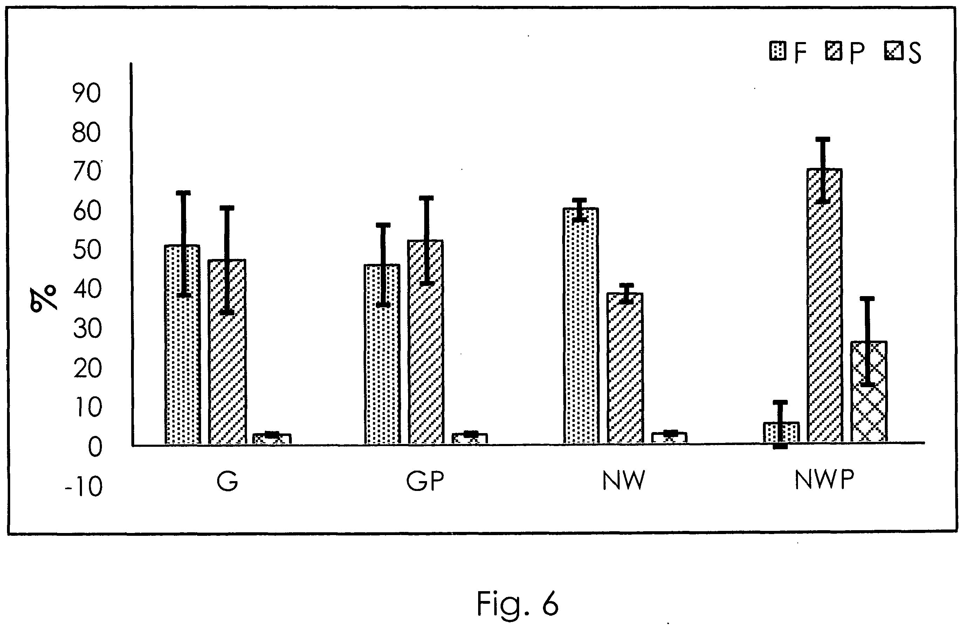

Darüber hinaus sind die Aktivierungsphasen der Thrombozyten auf den verschiedenen Proben untersucht worden. Dazu sind die Aktivierungsphasen unterteilt worden in nichtaktiviert (P) für Thrombozyten mit einer Oberfläche > 2 µm², teilaktiviert (S) für Thrombozyten mit einer Oberfläche zwischen 2 und 10 µm², und vollaktiviert (F) für Thrombozyten mit einer Oberfläche < 10 µm². Auch hier wurde die Berechnung der Oberfläche mit dem Programm Image J durchgeführt. Wie sich Fig. 6 entnehmen lässt, weist die erfindungsgemäße Beschichtung den geringsten Anteil von vollaktivierten und teilaktivierten Thrombozyten und den größten Anteil von nichtaktivierten Thrombozyten auch.

Zur Bestimmung der Zytotoxizität der Proben wurde Nährmedium mit menschlichen Kardiomyozyten (C-22070, PromoCell GmbH, Heidelberg, Germany) vorbereitet, nach weiterer Behandlung auf den Proben inkubiert und mittels eines LDH Assay (Cytotoxity Detection Kit, Roche Diagnostics) gemessen.

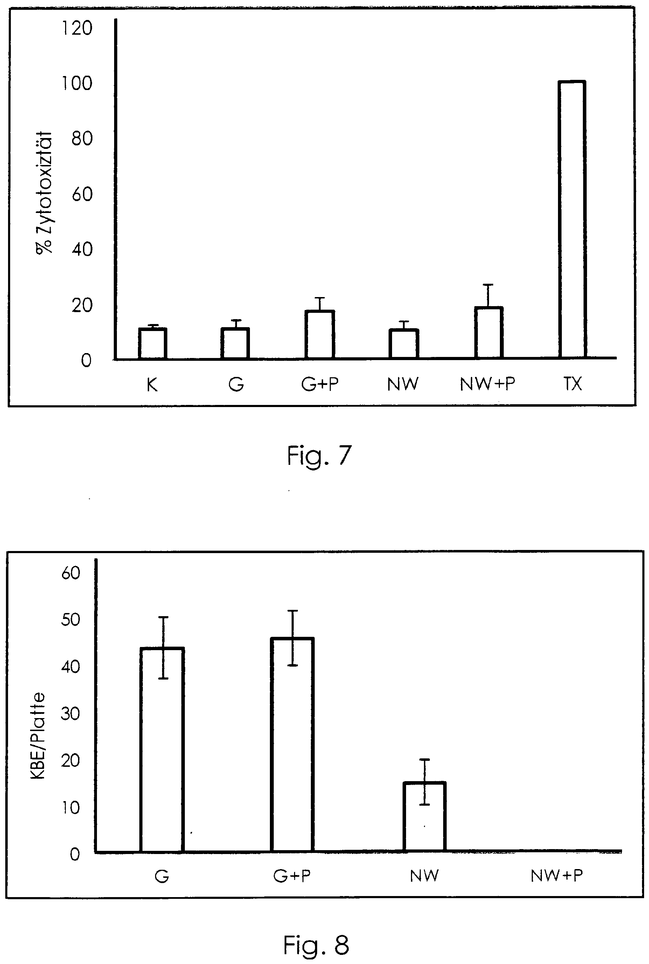

Als Referenz für minimale Zytotoxizität wurden von Behandlung freie Zellkulturen (K) verwendet. Zellen, die mit 2 % Triton X-100 behandelt worden waren, dienten als Referenz für 100 %ige Zytotoxizität. Die Messergebnisse an den Proben sind ins Verhältnis dazu gesetzt worden. Fig. 7 zeigt, dass die Proben der erfindungsgemäßen Beschichtung (NW+P) eine nur geringfügig größere Zytotoxizität aufweisen als die anderen Proben.

Um die Anlagerung von Bakterien auf den Proben zu untersuchen, wurden Proben per Agar in sterile 24 Well Platten (Greiner) fixiert und nachfolgend mit 1 ml PBS (Phosphat-gepufferte Salzlösung, pH 7,4) überschichtet. Für die Herstellung der Bakteriensuspension wurde, ausgehend von einer für 16 Stunden bei 37 °C inkubierten und mit dem Staphylococcus epidermidis Isolat RP62a beimpften Schafblutagarplatte, zwei bis drei Kolonien per Wattetupfer aufgenommen und in 2 ml PBS so lange eingerührt, bis eine McFarland-Trübung von 0,5 erreicht wurde. Diese Lösung wurde seriell in 10er Schritten in PBS bis zu einer Verdünnungsstufe von 10⁻⁴ verdünnt.

Die per Agar fixierten Oberflächen wurden nach Abnahme des PBS mit 1 ml der auf 10⁻⁴ verdünnten Bakterienlösung überschichtet und für 30 min bei 37 °C inkubiert. Nachfolgend wurde die Bakterienlösung vollständig abgesaugt und die Oberflächen zwei mal mit PBS gewaschen. Danach wurden die Oberflächen mit Hilfe einer sterilen Pinzette aus der 24 Well Platte herausgelöst und mit der Oberseite nach unten auf eine Luria-Bertani Agarplatte plaziert. Diese wurde für 24 Stunden bei 37 °C inkubiert und im Anschluss die Anzahl der koloniebildenden Einheiten (KBE) auf den Oberflächen bestimmt. Fig. 8 zeigt, dass die erfindungsgemäße Beschichtung die Anlagerung von Bakterienkolonien vollständig unterbunden hat. Bei den anderen Proben dagegen haben sich erhebliche Anzahlen von Bakterienkolonien gebildet.

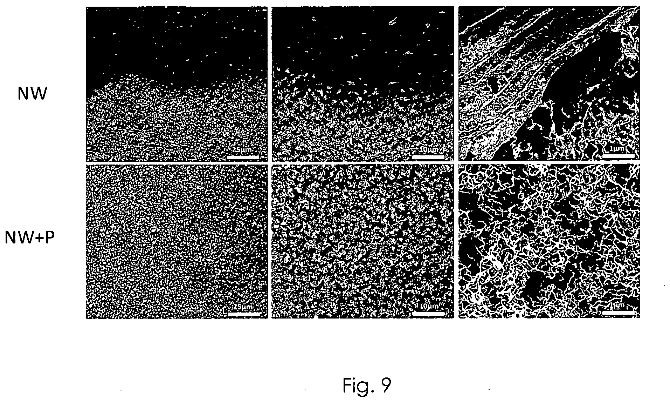

Zur Untersuchung der Proteinabsorption wurde die Proben mit 1ml menschlichem thrombopzytenarmen Plasma bei 37 °C während 30 min perfundiert. Um die Proben im Rasterelektronenmikrsokop untersuchen zu können, wurden die Proben, gewaschen, fixiert, kontrastiert und getrocknet. Die REM-Aufnahme in Fig. 9 zeigen, dass die Belegung der Nanodrähte mit PTFEP die Oberfläche dazu führt, dass eine Anlagerung von Proteinen vollständig verhindert wird.

## Patentansprüche

1. Beschichtung oder mit einer Beschichtung versehener Körper, wobei die Beschichtung zumindest teilweise aus einem Verbundwerkstoff gebildet ist, welcher einen porösen Träger umfasst, der einen metallischen Kern, welcher mit einem Metalloxid ummantelt ist und an dem ein Polyphosphazen gebunden ist, aufweist,
**dadurch gekennzeichnet,**
**dass** der Träger eine Vielzahl von Nanodrähten aufweist.

2. Beschichtung oder Körper nach Anspruch 1,
**dadurch gekennzeichnet,**
**dass** das Polyphosphazen chemisch, vorzugsweise über eine kovalente Bindung, an das Metalloxid gebunden ist.

3. Beschichtung oder Körper nach Anspruch 1 oder 2,
**dadurch gekennzeichnet,**
**dass** das Polyphosphazen ein Fluoro-Alkoxy-Phosphazen, vorzugsweise Poly-Trifluoroethoxy-Phosphazene (PTFEP), aufweist.

4. Beschichtung oder Körper nach einem der Ansprüche 1 bis 3,
**dadurch gekennzeichnet,**
**dass** das Poly-Trifluoroethoxy-Phosphazene vorliegt wie folgt:

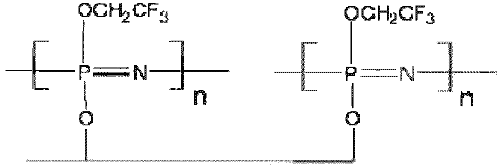

5. Beschichtung oder Körper nach einem der Ansprüche 1 bis 4,
**dadurch gekennzeichnet,**
**dass** die Nanodrähte untereinander vernetzt sind.

6. Beschichtung oder Körper nach einem der Ansprüche 1 bis 5,
**dadurch gekennzeichnet,**
**dass** das Metall des Metalloxids und das Metall des Kerns ein Metall der III. Hauptgruppe sind.

7. Beschichtung oder Körper nach einem der Ansprüche 1 bis 6,
**dadurch gekennzeichnet,**
**dass** das Metall des Kerns Aluminium oder Gallium ist und das Metalloxid Aluminiumoxid oder Galliumoxid ist.

8. Beschichtung oder Körper nach einem der Ansprüche 1 bis 7,
**dadurch gekennzeichnet,**
**dass** das Polyphosphazen den Träger, insbesondere die Nanodrähte, mit einer derart dünnen Schicht belegt, dass zumindest an der Oberfläche der Beschichtung die durch die Nanodrähte gebildete Struktur erhalten bleibt.

9. Beschichtung oder Körper nach einem der Ansprüche 1 bis 8,
**dadurch gekennzeichnet,**
**dass** der Körper ein Implantat, vorzugsweise ein Stent oder eine Gelenkersatzimplantat, eine Glasscheibe, vorzugsweise einer Fensterscheibe, eine Sanitärobjekt, vorzugsweise ein WC-Becken, ein Urinal, eine Badewanne oder eine Duschtasse, ist.

10. Verfahren zur Herstellung einer Beschichtung oder eines mit der Beschichtung versehenen Körpers, wobei die Beschichtung zumindest teilweise aus einem Verbundwerkstoff gebildet wird, welcher einen porösen Träger, der einen metallischen Kern, welcher mit einem Metalloxid ummantelt ist, aufweist, wobei an den Träger ein Polyphosphazen gebunden wird,
**dadurch gekennzeichnet,**
**dass** der Träger aus Nanodrähten hergestellt wird.

11. Verfahren nach Anspruch 10,
**dadurch gekennzeichnet,**
**dass** das Polyphosphazen chemisch, vorzugsweise über eine kovalente Bindung, an das Metalloxid gebunden wird.

12. Verfahren nach Anspruch 10 oder 11,
**dadurch gekennzeichnet,**
**dass** Fluoro-Alkoxy-Phospazen, vorzugsweise Poly-Trifluoroethoxy-Phosphazene (PTFEP) an den Träger gebunden wird.

13. Verfahren nach einem der Ansprüche 10 bis 12,
**dadurch gekennzeichnet,**
**dass** das Polyphosphazen mittels Dip-Coating auf den Träger aufgebracht wird, wobei das Polyphosphazen beim Dip-Coating vorzugsweise in einer Konzentration von 0,2 bis 3,5 mg/cm³, besonders bevorzugt in einer Konzentration von 0,5 bis 2,5 mg/cm³, vorliegt.

## Claims

1. Coating, or body provided with a coating, where the coating is formed at least partly of a composite material comprising a porous carrier which comprises a metallic core that is clad with a metal oxide and that has a polyphosphazene bonded on it,
**characterized in that**
the carrier comprises a multiplicity of nanowires.

2. Coating or body according to Claim 1,
**characterized in that**
the polyphosphazene is bonded chemically, preferably via a covalent bond, to the metal oxide.

3. Coating or body according to Claim 1 or 2,
**characterized in that**
the polyphosphazene comprises a fluoro alkoxy phosphazene, preferably poly trifluoroethoxy phosphazene (PTFEP).

4. Coating or body according to any of Claims 1 to 3,
**characterized in that** the poly trifluoroethoxy phosphazene is present as follows:

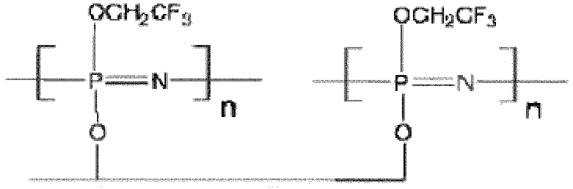

5. Coating or body according to any of Claims 1 to 4,
**characterized in that**
the nanowires are linked with one another.

6. Coating or body according to any of Claims 1 to 5,
**characterized in that**
the metal of the metal oxide and the metal of the core is a metal of main group III.

7. Coating or body according to any of Claims 1 to 6,
**characterized in that**
the metal of the core is aluminium or gallium and the metal oxide is aluminium oxide or gallium oxide.

8. Coating or body according to any of Claims 1 to 7,
**characterized in that**
the polyphosphazene covers the carrier, more particularly the nanowires, with a layer so thin that at least at the surface of the coating, the structure formed by the nanowires is retained.

9. Coating or body according to any of Claims 1 to 8,
**characterized in that**
the body is an implant, preferably a stent or joint replacement implant, a glass sheet, preferably a window sheet, a sanitary object, preferably a toilet basin, a urinal, a bathtub or a shower tray.

10. Method for producing a coating or a body provided with the coating, where the coating is formed at least partly of a composite material comprising a porous carrier which comprises a metallic core that is clad with a metal oxide wherein a polyphosphazene is bonded on the carrier,
**characterized in that**
the carrier is produced from nanowires.

11. Method according to Claim 10,
**characterized in that**
the polyphosphazene is bonded chemically, preferably via a covalent bond, to the metal oxide.

12. Method according to Claim 10 or 11,
**characterized in that**
fluoro alkoxy phosphazene, preferably poly trifluoroethoxy phosphazene (PTFEP), is bonded to the carrier.

13. Method according to any of Claims 10 to 12,
**characterized in that**
the polyphosphazene is applied to the carrier by means of dip coating, where the polyphosphazene during the dip coating is preferably at a concentration of 0.2 to 3.5 mg/cm³, more preferably at a concentration of 0.5 to 2.5 mg/cm³.

## Revendications

1. Revêtement ou corps pourvu d'un revêtement, le revêtement étant formé au moins partiellement à partir d'un matériau composite, qui comprend un support poreux qui présente un noyau métallique enveloppé, par un oxyde métallique, et auquel un polyphosphazène est lié, **caractérisé en ce que** le support présente une multitude de nanofils.

2. Revêtement ou corps selon la revendication 1, **caractérisé en ce que** le polyphosphazène est lié de manière chimique, de préférence par l'intermédiaire d'une liaison covalente, à l'oxyde métallique.

3. Revêtement ou corps selon la revendication 1 ou 2, **caractérisé en ce que** le polyphosphazène présente un fluoro-alcoxy-phosphazène, de préférence un poly-trifluoroéthoxy-phosphazène (PTFEP).

4. Revêtement ou corps selon l'une quelconque des revendications 1 à 3, **caractérisé en ce que** le poly-trifluoroéthoxy-phosphazène se présente comme suit :

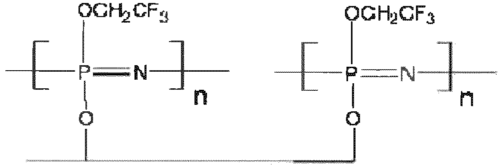

5. Revêtement ou corps selon l'une quelconque des revendications 1 à 4, **caractérisé en ce que** les nanofils sont reliés entre eux.

6. Revêtement ou corps selon l'une quelconque des revendications 1 à 5, **caractérisé en ce que** le métal de l'oxyde métallique et le métal du noyau sont un métal du Illème groupe principal.

7. Revêtement ou corps selon l'une quelconque des revendications 1 à 6, **caractérisé en ce que** le métal du noyau est l'aluminium ou le gallium et l'oxyde métallique est l'oxyde d'aluminium ou l'oxyde de gallium.

8. Revêtement ou corps selon l'une quelconque des revendications 1 à 7, **caractérisé en ce que** le polyphosphazène recouvre le support, en particulier les nanofils, en une couche tellement mince que la structure formée par les nanofils reste conservée, au moins à la surface du revêtement.

9. Revêtement ou corps selon l'une quelconque des revendications 1 à 8, **caractérisé en ce que** le corps est un implant, de préférence un stent ou un implant de prothèse articulaire, une feuille de verre, de préférence une vitre, un objet sanitaire, de préférence une cuvette de WC, un urinoir, une baignoire ou un receveur de douche.

10. Procédé pour la réalisation d'un revêtement ou d'un corps pourvu d'un revêtement, le revêtement étant formé au moins partiellement à partir d'un matériau composite, qui comprend un support poreux, qui présente un noyau métallique, qui est enveloppé par un oxyde métallique, un polyphosphazène étant lié au support, **caractérisé en ce que** le support est fabriqué à partir de nanofils.

11. Procédé selon la revendication 10, **caractérisé en ce que** le polyphosphazène est lié de manière chimique, de préférence par l'intermédiaire d'une liaison covalente, à l'oxyde métallique.

12. Procédé selon la revendication 10 ou 11, **caractérisé en ce que** du fluoro-alcoxy-phosphazène, de préférence du poly-trifluoroéthoxy-phosphazène (PTFEP) est lié au support.

13. Procédé selon l'une quelconque des revendications 10 à 12, **caractérisé en ce que** le polyphosphazène est appliqué sur le support par revêtement par immersion, où, lors du revêtement par immersion, le polyphosphazène se trouve de préférence en une concentration de 0,2 à 3,5 mg/cm³, de manière particulièrement préférée en une concentration de 0,5 à 2,5 mg/cm³.
